# EUROPÄISCHE PATENTSCHRIFT

(11) **EP 3 033 116 B1**
(45) Veröffentlichungstag und Bekanntmachung des Hinweises auf die Patenterteilung: **07.04.2021**
(21) Anmeldenummer: 14750375.9
(22) Anmeldetag: 12.08.2014
(51) Int. Cl.: A61L 15/28, A61L 15/46, A61L 15/58, A61L 15/60, C08L 33/08, C08L 33/10

(54) **WUNDPFLEGEARTIKEL AUFWEISEND SUPERABSORBIERENDE FASERN UND SUPERABSORBIERENDE PARTIKEL**
WOUND CARE ARTICLE HAVING SUPER-ABSORBENT FIBERS AND SUPER-ABSORBENT PARTICLES
ARTICLE POUR LE SOIN DE PLAIES, PRÉSENTANT DES FIBRES SUPER-ABSORBANTES ET DES PARTICULES SUPER-ABSORBANTES

(30) Priorität: 12.08.2013 DE 102013108734; 31.10.2013 DE 202013104893 U
(43) Veröffentlichungstag der Anmeldung: 22.06.2016
(73) Patentinhaber: BSN medical GmbH, 22761 Hamburg (DE)
(72) Erfinder: RIESINGER, Birgit, 48149 Münster (DE)
(74) Vertreter: FARAGO Patentanwälte
(86) Internationale Anmeldenummer: PCT/EP2014/067282
(87) Internationale Veröffentlichungsnummer: WO 2015/022340

(56) Entgegenhaltungen:
- WO-A1-2013/026879
- WO-A1-2013/026912

## Beschreibung

Die vorliegende Erfindung betrifft Wundpflegeartikel aufweisend mindestens eine flächige Lage aus einem Gewebe oder einem Vlies aufweisend Superabsorbierende Fasern, und Superabsorbierende Partikel, wobei die flächige Lage ferner Bi-Component-Fasern aufweist, und wobei die Bi-Component-Fasern einen Anteil eines thermoplastischen Polymers mit vergleichsweise niedrigem Schmelzpunkt enthalten, das bei Erwärmen schmilzt und so als Heißkleber fungiert.

Wundpflegeartikel aufweisend Superabsorbierende Polymere sind beispielsweise aus der EP1507498 bekannt und haben sich in der Versorgung chronischer und stark exsudierender Wunden sehr bewährt. In diesen Wundpflegeartikeln sind superabsorbierende Polymer-Partikel in eine Zellstoff-Matrix eingebracht.

Für bestimmte Einsatzgebiete und Indikationen kann sich der hierdurch bedingte relativ geringe Gewichtsanteil an Superabsorbierenden Polymeren (obwohl teils über 60 Gew.-%) als nachteilig erweisen.

Auch weisen die herkömmlichen Wundauflagen eine gewisse Steifigkeit und geringe Flexibilität auf, was beispielsweise für tiefe Wunden nachteilig ist, weil u.U. ein Kontakt zum Wundgrund nicht immer gewährleistet werden kann. Diese Steifigkeit ist nicht zuletzt durch die oftmals sehr hohe Dichte solcher Wundauflagen, bzw. deren Innenlagen, bedingt.

Ebenso kann bei den herkömmlichen Wundauflagen unter gewissen Umständen die aufgenommene Flüssigkeit horizontal verteilt werden, was die Gefahr von Wundrandmazeration birgt.

WO2013/026912 A offenbart einen Wundpflegeartikel aufweisend einen Anteil an hydroaktiven Polymer (besonders superabsorbierende Polymere) und einen Anteil an Kupfer bzw. Kupferionen. In einer Ausführungsform enthält der Absorptionskörper eine flache Lage aufweisend Fasern oder Garne aus superabsorbierenden Polymeren, an welche superabsorbierende Polymere in Granulatform geklebt sind.

Aufgabe der vorliegenden Erfindung ist es, einen Wundpflegeartikel bereitzustellen, der diese Nachteile nicht aufweist.

Diese Aufgabe wird mit den Merkmalen des vorgelegten Hauptanspruchs gelöst. Gegenstand der Erfindung ist ein Wundpflegeartikel aufweisend mindestens eine flächige Lage aus einem Gewebe oder einem Vlies aufweisend
- Superabsorbierende Fasern, und
- Superabsorbierende Partikel,
- wobei die flächige Lage ferner Bi-Component-Fasern aufweist, und wobei die Bi-Component-Fasern einen Anteil eines thermoplastischen Polymers mit vergleichsweise niedrigem Schmelzpunkt enthalten, das bei Erwärmen schmilzt und so als Heißkleber fungiert.

Der Begriff "Wundpflegeartikel" soll im Folgenden insbesondere eine Wundauflage, bevorzugt eine flächige Wundauflage oder ein Wundpflegetuch bezeichnen. Besagte Wundauflage enthält eine flächige Lage und kann dabei sowohl absorbierend als auch nicht oder nur unwesentlich absorbierend ausgestaltet sein. Insbesondere kann der Begriff "Wundpflegeartikel" auch als ein Ensemble verschiedener Produkte verstanden werden, die in einer gegebenen Anordnung auf der zu behandelnden Wunde angeordnet werden. Dieses Ensemble kann eine physikalische Einheit bilden, indem die verschiedenen Produkte in einer gemeinsamen Hülle zusammengefasst oder - ggf. ohne Hülle - adhäsiv miteinander verbunden sind. Das Ensemble kann jedoch auch in Form eines Kits vorliegen, bei dem die verschiedenen Produkte mithilfe einer Wickel in der gegebenen Anordnung auf der zu behandelnden Wunde angeordnet sind.

Der Begriff "Vlies" bezeichnet ein textiles Flächengebilde aus einzelnen Fasern, das im Gegensatz zu Geweben, Gestricken und Gewirken nicht aus Garnen hergestellt wird. Vliese behalten ihre strukturelle Integrität i.d.R. durch Haftung der einzelnen Fasern aneinander. Sie werden auch als "Nonwovens" bezeichnet, und z.B. durch Walken der Fasern hergestellt. Der Begriff "Airlaid" bezeichnet einen speziellen Vliesstoff aus Zellstoff und Polyolefinfasern, in den ggf. superabsorbierende Polymere eingebettet sind.

Der Begriff "Exsudat" bezeichnet eine über entzündliche Prozesse vom Blutplasma abgeleitete Wundflüssigkeit. So wie das Blut für den Transport von Nährstoffen und anderen Botenstoffen und damit für die Versorgung verschiedener Teile des Körpers verantwortlich ist, dient das Exsudat auf ganz ähnliche Weise der Versorgung des Wundbettes und der darin ablaufenden Heilungsprozesse. Um dieser Vielzahl an Funktionen gerecht zu werden, enthält es ein breites Spektrum an Komponenten, woraus ein spezifisches Gewicht resultiert, das leicht oberhalb dessen von Wasser liegt. Darin unterscheidet es sich auch vom Transsudat, welches von nicht-entzündlichen Prozessen abgeleitet ist und ein deutlich geringeres spezifisches Gewicht mit einem geringen Zell- und Proteingehalt aufweist. Neben der Bereitstellung von Nährstoffen für die Fibroblasten und Epithelzellen beeinflusst die Beschaffenheit des Exsudats die verschiedenen Prozesse der Wundheilung zeitlich und räumlich durch seinen hohen Gehalt an Wachstumsfaktoren und Zytokinen. Diese werden vor allem durch Thrombozyten, Keratinozyten, Makrophagen und Fibroblasten gebildet. Sie beeinflussen die Motilität, Migration und Proliferation der verschiedenen an der Wundheilung beteiligten Zellen. So wird das Einwandern von Zellen in den Wundgrund ebenso gefördert, wie die Versorgung des neugebildeten Granulationsgewebes durch die Angiogenese. Auch die Wundreinigung wird durch das Exsudat unterstützt. Es enthält verschiedene Serin-, Cystein-und Aspartatproteasen sowie Matrix-Metalloproteasen, die in ihrer Aktivität streng reguliert irreversibel geschädigtes Gewebe abbauen und somit das Wundbett für die nachfolgenden Phasen der Heilung vorbereiten. Generell unterscheidet man bei solchen Prozessen zwischen physiologischem und pathologischem Exsudat.

Bestandteile des physiologischen Exsudats sind insbesondere Salze, Glucose, Zytokine und Wachstumsfaktoren, Plasmaproteine, Proteasen (insbesondere Matrix-Metalloproteasen), Granulozyten und Makrophagen.

Durch die Fasereigenschaften vereint der erfindungsgemäße Wundpflegeartikel die Eigenschaften drei verschiedener, heute auf dem Markt befindlicher Kategorien von Wundpflegeartikeln:
- Er weist eine ähnlich hohe Absorptionskapazität auf wie Wundpflegeprodukte, die eine Airlaid-Matte aufweisend ein Zellstoff-Vlies mit eingebetteten Superabsorbierenden Partikeln auf (Beispiel: Produkt "sorbion sachet" der Sorbion GmbH & Co. KG), ist daher auch für stark exsudierende Wunden sowie solche Wunden, die durch ein tiefliegendes Ödem verursacht werden, geeignet
- Er weist eine ähnlich hohe Oberflächenfeuchte und Polsterung wie ein Schaumstoffverband auf (Beispiel: Produkt "Allevyn Adhesive Foam" von Smith & Nephew), weist daher eine geringere Neigung zur Trocknung der Wunde sowie ein hohes Komfortniveau für den Patienten auf. Insbesondere der fluffige, viel Luft umschließende Faseraufbau ist für die Weichheit verantwortlich
- Er weist ebenso wie ein Verband aufweisend Fasern aus Carboxymethylcellulose ("Hydrofaser", Beispiel: Produkt "Aquacel" von ConvaTec) eine vertikale Aufnahme von Flüssigkeit verbunden mit einer stark verminderten lateralen Verteilung von Flüssigkeit auf, sodass (i) Wundrandmazeration verhindert wird und (ii) der Artikel in der Lage ist, sich einer Wundkontur anzupassen ("Macrocontouring") .

Ein solches Produkt aufweisend einen erfindungsgemäßen Wundpflegeartikel ist beispielsweise unter der Bezeichnung "sorbion soft" im Handel.

Bevorzugt ist vorgesehen, dass die Superabsorbierenden Fasern ein quervernetzes Polymer aufweisen, das mindestens aus den Monomerbestandteilen
- Acrylsäure bzw. Acrylat ("AA"), partiell zum Natriumsalz neutralisert ("AA- Na"),
- Methylacrylat bzw. Methylacrylsäure ("MA"), sowie
- spezielles Acrylat/Methylacrylat Monomer ("SAMM")
hergestellt ist, wobei die Quervernetzungen zwischen den einzelnen Polymerketten als Esterbindungen zwischen den Säuregruppen der Acrylsäure bzw. dem Methylacrylat und dem partiellen Natriumsalz ausgebildet sind.

Die generelle Strukturformel sieht wie folgt aus:

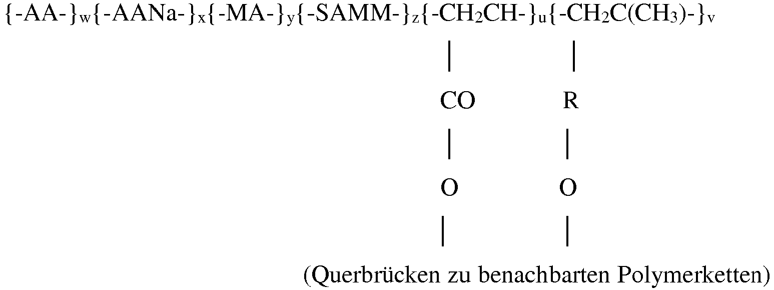

Dabei sind:

| | |
|---|---|
| AA | CH₂CH(COOH)⁻ |
| MA | CH₂CH(COOCH₃)- |
| AANa | CH₂CH(COONa)⁻ |
| R | CH₂CH(CH₃)⁻ |

Beim dem speziellen Acrylat/Methylacrylat Monomer ("SAMM") handelt es sich bevorzugt um ein Monomer ausgewählt aus der Gruppe enthaltend
- Hexapropylen Glycol Monomethacrylat
- 2-Hydroxyethyl(meth)acrylat
- Polyethylene Glycol Monomethacrylat
- Glycidyl Methacrylat
- Allyl Glycidyl Ether,
- Hydroxypropyl Methacrylat, und/oder
- Hydroxyethyl Methacrylat

Von diesen Substanzen ist Hexapropylen Glycol Monomethacrylat besonders bevorzugt.

Die Werte von W, X, Y und Z bestimmen die Anteile der Ausgangsmaterialien, während die Werte von U und V abhängig sind vom der Temperatur beim Herstellungsprozess.

Die quantitative Zusammensetzung kann bevorzugt wie folgt sein (Angaben in Mol%):

| **Bestandteil** | **Bevorzugt** | **Sehr bevorzugt** | **Besonders bevorzugt** |
|---|---|---|---|
| AA | 30-80 | 40-70 | 58,5 |
| AANa | 10-30 | 15-25 | 19,5 |
| MA | 10-30 | 15-25 | 20 |
| SAMM | 0,5-4 | 1 - 3 | 2 |

Die besagten Fasern können mit den anderen genannten fasern zu einem Airlaid ausgebildet sein. Der Begriff "Airlaid" bezeichnet einen speziellen Vliesstoff, in den ggf. superabsorbierende Polymere eingebettet sind. Dabei kommen bevorzugt folgende dem Fachmann prinzipiell bekannte Technologien zum Einsatz:
- MBAL = Multi-Bonded Air-Laid
- BBAL/LBAL = Binder-Bonded Air-Laid/Latex-Bonded Air-Laid
- HBAL/XBAL = Hydrogen-Bonded Air-Laid/X-Bonded Air-Laid
- Spun bound-Verfahren
- Wet laid-Verfahren

"Superabsorbierende Partikel" (SAP) sind Kunststoffe, die in der Lage sind, ein Vielfaches ihres Eigengewichts - bis zum 1000-fachen - an Flüssigkeiten aufzusaugen. Chemisch handelt es sich dabei um ein Copolymer aus Acrylsäure (Propensäure, C₃H₄O₂) und Natriumacrylat (Natriumsalz der Acrylsäure, NaC₃H₃O₂), wobei das Verhältnis der beiden Monomere zueinander variieren kann. Zusätzlich wird ein so genannter Kernvernetzer (Core-Cross-Linker, CXL) der Monomerlösung zugesetzt, der die gebildeten langkettigen Polymermoleküle stellenweise untereinander durch chemische Brücken verbindet (sie "vernetzt"). Durch diese Brücken wird das Polymer wasserunlöslich. Beim Eindringen von Wasser oder wässrigen Salzlösungen in den Polymerpartikel quillt er auf und strafft auf molekularer Ebene dieses Netzwerk, so dass das Wasser ohne Hilfe nicht mehr entweichen kann.

Alternativ können die Superabsorber auf Methylacrylsäurebasis, Polyvinylalkohol-Maleinsäureanhydrid-Copolymere, Polysaccharid-Maleinsäureanhydrid-Copolymere, Maleinsäurederivate, Acrylamidopropansulfonsäure-Copolymere, Stärke-Acrylnitril-Pfropfpolymere, gelatinisierte Stärkederivate, Alkyl- oder Hydroxyalkylcellulose, Carboxymethylcellulose, Stärke-Acrylsäure-Pfropfpolymere, Vinylacetat-Acrylsäure-ester-Copolymere, Acrylnitril- oder Acrylamid-Copolymere gewählt sein.

Die Superabsorbierenden Partikel können in Pulver- oder Granulatform in einer Partikelgröße zwischen 100 und etwa 1000 µm vorliegen.

Ebenso kann es sich bei besagten superabsorbierenden Polymeren auch um Hydrogel-Nanopartikel aufweisend Hydroxy-Terminierte Methacrylatmonomere, wie 2-Hydroxyethylmethacrylat (HEMA) und/oder 2-Hydroxypropylmethacrylat (HPMA), die z.B. als Altrazeal vermarktet werden, handeln.

Der betreffend ausgerüstete Wundpflegeartikel weist eine Vielzahl von Vorteilen auf.

Die Kombination der verschiedenen superabsorbierenden Materialien ermöglicht eine schnelle Aufnahme von Flüssigkeiten ohne eine horizontale Ausleitung der Flüssigkeit in die breite. Dabei wird einerseits - ganz wie bei Carboxymethylzellulose - eine gelfeuchte Wundkontaktoberfläche generiert, die u.U. eine kühlende Wirkung ausübt und ein heilungsförderndes Milieu erzeugt, durch das Ausbleiben der horizontalen Ausleitung aber gleichzeitig ein Feuchtkontakt mit den Wundrändern und die damit verbundene Wundrandmazeration aber verhindert.

Weiterhin weist der erfindungsgemäße Wundpflegeartikel ohne dass weitere Behandlungsschritte notwendig wären, bereits eine große Weichheit und Anschmiegsamkeit und einen angenehmen Griff auf, die vom Patienten als subjektiv angenehm empfunden werden, aber auch objektiv große Vorteile bieten, da sie einer Traumatisierung beim Verbandwechsel entgegenwirken, eine Anpassung an die Wundkontur erlauben und gleichzeitig das Schmerzempfinden beim Patienten reduzieren. Hinzu kommen polsternde Eigenschaften, die insbesondere bei einer Kompressions- oder Vakuumtherapie vorteilhaft zum Tragen kommen. Außerdem kann der Wundpflegeartikel auf diese Weise auch Gewebelücken oder Substanzdefekte ausfüllen ("Wundeinlage"), wobei in einem solche Fall vorgesehen kann, dass der Wundpflegeartikel nicht über den Wundrand lappt.

Insbesondere die Anpassung an die Wundkontur ist besonders vorteilhaft, weil auf diese Weise sichergestellt werden kann, dass der Wundpflegeartikel direkten Kontakt zum Wundgrund und dem dort vorhandenen Exsudat herstellen kann. Damit ist eine zügige Aufnahme und Ableitung des Exsudats gewährleistet.

Weiterhin weist der erfindungsgemäße Wundpflegeartikel starke antimikrobielle Eigenschaften auf, die einerseits durch die Eigenschaft der Superabsorber, Proteine und Bakterien zu binden, bedingt sind, und andererseits auf deren wasserbindende Eigenschaften zurückzuführen sind, durch welche den Bakterien die für die Aktivität notwendige Flüssigkeit entzogen wird.

Darüber hinaus ist gewährleistet, dass das Produkt auch nach Aufnahme großer Mengen Exsudat seine strukturelle Integrität behält und im Ganzen von der Wunde entfernt werden kann.

Weiterhin konnte gezeigt werden, dass die Kombination der verschiedenen superabsorbierenden Materialien eine Modulation von proinflammatorischen Faktoren, wie Matrix-metallo-Proteasen ("MMPs"), Sauerstoffradikale ("ROS"), ILlbeta, IL6, IL8 und TNF alpha ermöglicht. Auch dieser Effekt ist auf die bindenden Eigenschaften der superabsorbierenden Polymere gegenüber Proteinen zurückzuführen.

Ferner kommt einem solchen Produkt auch eine Belagauflösende Wirkung zu. Dies betrifft insbesondere Biofilme und fibrinöse Beläge.

Aufgrund der genannten Eigenschaften erschließen sich dem erfindungsgemäßen Wundpflegeartikel eine Reihe neuer Indikationen. Insbesondere eignet sich der erfindungsgemäße Wundpflegeartikel für akute und postinterventionelle Wunden, chronische Wunden, wie zum Beispiel diabetische Wunden oder Druckgeschwüre, Tumorwunden, Verbrennungen, leicht bis stark exsudierende Wunden, sowie tiefe Wunden, die gegebenenfalls eine Defektauffüllung benötigen.

Erfindungsgemäß ist vorgesehen, dass die flächige Lage ferner Bi-Component-Fasern aufweist.

Besonders bevorzugt ist vorgesehen, dass die flächige Lage ferner Zellulosefasern aufweist.

Besagte Zellulosefasern können bevorzugt als sogenanntes Fluff-Pulp vorliegen, und haben neben polsternden auch flüssigkeitsbindende sowie strukturerhaltende Eigenschaften.

Bei den Bi-Component-Fasern handelt es sich erfindungsgemäß um Fasern, die ein Thermobonding der flächigen Lage erleichtern. Sie enthalten daher einen Anteil eines thermoplastischen Polymers mit vergleichsweise niedrigem Schmelzpunkt, beispielsweise aus Polyester, Polypropylen oder Polyethylen, das bei Erwärmen schmilzt und so als Heißkleber (Hotmelt) fungiert.

Dies ist auch deswegen vorteilhaft, weil auf diese Weise das für das herkömmliche Hydrogenbonding, wie es in Airlaids ohne diese Fasern zur Anwendung kommt, notwendige Pressen der Matte bei hohem Druck, Wärme und Luftfeuchtigkeit (beispielsweise durch Kalandrieren) wird sichtbar macht. Letzteres führt zu einer hohen Verdichtung der Matte und beeinträchtigt auf diese Weise die Bauschigkeit. Die Verwendung besagter Fasern erhöht also den Lufteinschluss in der Matte, was wiederum zu einer schnelleren Aufnahme der Wundflüssigkeit durch sich einstellende Kapillareffekte führt.

Besagte Bi-Component-Fasern werden bevorzugt durch Coextrusion zweier Polymere mit unterschiedlichen Physikochemischen Eigenschaften erzeugt. Dabei kommen bevorzugt die "sheath/core-Konfiguration", bei welcher die niedrigschmelzende Komponente aussen und die hochschmelzende Komponente innen liegt, und die "side-by-side Konfiguration", bei welche die beiden Komponenten im Querschnitt der Faser halbkreisförmig zueinander angeordnet sind, zum Einsatz.

| **Beispiel** | **Sheath** | **Core** |
|---|---|---|
| 1 | Copolyester (Schmelzpunkt 110-120°C | Polyester (Schmelzpunkt 250 °C) |
| 2 | Polyethylene (Schmelzpunkt 130 °C) | Polyester (Schmelzpunkt 250 °C) |
| 3 | Polyethylene (Schmelzpunkt 130 °C) | Polypropylen (Schmelzpunkt 175 °C) |

Alternativ kann vorgesehen sein, dass die Bi-Component-Fasern neben einem thermoplastischen Polymer einen Compound aus mindestens einem thermoplastischen Basispolymer und mindestens einem superabsorbierenden Polymer (SAP) enthalten. Dabei ist bevorzugt der Schmelzpunkt des erstgenannten Thermoplasten um mindestens 20°C höher ist als der Schmelzpunkt des in dem Compound enthaltenen Thermoplasten.

Die folgende Tabelle zeigt beispielhafte Zusammensetzungen einer bevorzugten flächigen Lage. Dabei sind die Wertebereiche einschließlich der die Wertebereiche eingrenzenden Zahlenwert zu verstehen.

| **Bestandteil** | **Bevorzugt** | **Sehr bevorzugt** | **Besonders bevorzugt** |
|---|---|---|---|
| Zellulosefasern | 10 - 35 Gew.-% | 15 - 30 Gew.-% | 20 - 25 Gew.-% |
| Bi-Componentfasern | 0,5 - 15 Gew.-% | 1-10 Gew.-% | 3-8 Gew.-% |
| Superabsorbierende Fasern | 15-60 Gew-% | 20-50 Gew-% | 30- 40 Gew-% |
| Superabsorbierende Partikel | 15 -60 Gew-% | 20-50 Gew-% | 30- 40 Gew-% |

Grundsätzlich kann festgestellt werden, dass Zellulosefasern Flüssigkeit sehr schnell aufnehmen, demgegenüber aber eine relativ geringe Absorptionskapazität aufweisen und auch keinen Dochteffekt aufweisen.

Bi-Componentfasern hingegen nehmen praktisch keine Flüssigkeit auf, weisen demgegenüber aber einen hohen Dochteffekt auf.

Superabsorbierende Fasern benötigen einige Zeit, um Flüssigkeit aufzunehmen, weisen dann jedoch eine sehr hohe Absorptionskapazität auf. In der Anfangsphase weisen sie einen sehr hohen Dochteffekt auf, der mit Beginn des Absorptionsprozesses hingegen rapide abnimmt.

Durch gezielte Wahl der quantitativen Faserzusammensetzung kann das Absorptions- und Flüssigkeitsverteilungsverhalten des erfindungsgemäßen Wundpflegeartikels in den einzelnen Schichten damit sehr genau gesteuert werden.

So kann beispielsweise die äußeren Schichten so eingestellt werden, dass sie einen geringen Dochteffekt aufweisen, um beispielsweise Wundrandmazeration zu vermeiden, während innenliegende Schichten so eingestellt werden können, dass sie einen hohen Dochteffekt aufweisen, um eine großflächige Verteilung der aufgenommenen Flüssigkeit in der Fläche zu ermöglichen.

Im Folgenden sind die Begriffe "Geringer Dochteffekt", "Mittelgradiger Dochteffekt" und "Hoher Dochteffekt" exemplarisch als sogenannte "vertical wicking height [cm/h]" quantifiziert:

| **Geringer Dochteffekt** | **Mittelgradiger Dochteffekt** | **Hoher Dochteffekt** |
|---|---|---|
| 0,1-1cm/h | 1-5 cm/h | 10 - 100 cm/h |

Die folgende Tabelle zeigt beispielhafte Eigenschaften einer bevorzugten flächigen Lage. Dabei sind die Wertebereiche einschließlich der die Wertebereiche eingrenzenden Zahlenwert zu verstehen.

| **Parameter** | **Bevorzugt** | **Sehr bevorzugt** | **Besonders bevorzugt** |
|---|---|---|---|
| Flächengewicht (g/m²) | 100-900 | 450-750 | 550-600 |
| Dicke (mm) | 1-10 | 2-8 | 3,5 - 4,5 |
| Aufnahmekapazität 0.9% Kochsalzlösung (g/g) | 10 - 100 | 20 - 70 | 30 - 40 |
| Aufnahmekapazität demineralisiertes Wasser (g/g) | 40 - 400 | 60 - 200 | 80 - 100 |

Dabei ist bevorzugt vorgesehen, dass die flächige Lage im Randbereich dünner ausfällt, sich also beispielsweise im Querschnitt an den Rändern konisch verjüngt. Damit ist gewährleistet, dass an den Wundrändern ein geringerer Auftrag vorliegt.

Ferner ist bevorzugt vorgesehen, dass die flächige Lage mindestens auf einer Seite von einem dünnen Nonwoven be- oder unterlegt ist. Hierbei kann es sich beispielsweise um ein dünnes, wasserdurchlässiges Vlies aus Polypropylen, Polyethylen oder Polyester handeln. Ebenso kann dieses Nonwoven aus einem Zellulosevlies bestehen.

Grundsätzlich kann vorgesehen sein, dass die genannten Nonwoven insbesondere dann, wenn sie aus einem tendenziell hydrophoben Material bestehen, durch eine sogenannte Avivage behandelt werden, die zu einer verbesserten Benetzbarkeit führt und somit den flüssigkeitsdurchtritt erleichtert. Diese Avivage wird jedoch u.U. durch den anschließenden Kalandrierungsprozess beeinträchtigt.

Da beim Airlaid-Prozess Fasern von oben auf das Nonwoven aufgeblasen werden, wird sich im Anfangsstadium ein Zustand einstellen, bei welchem einige Fasern das Nonwoven senkrecht durchdringen. Dieser Zustand kann wünschenswert sein, weil diese Fasern den Flüssigkeitseintritt in die Matte erleichtern, und damit die oben genannte Beeinträchtigung der Avivage mindestens teilweise kompensieren.

Allerdings gilt auch hier, dass eine möglichst ideale Symmetrie des Schichtaufbaus bevorzugt ist. Wird, nachdem mehrere Faserlagen in den Herstellungsverfahren aufgebracht worden sind, mit einem oberen abschließenden Nonwoven gearbeitet, besteht das Risiko, dass die letzte genannte Schicht nicht durch senkrecht stehende Fasern durchdrungen wird. Dies kann dazu führen, dass das besagte obere abschließende Nonwoven der Flüssigkeit einen höheren Widerstand entgegensetzt als das untere Nonwoven. Aus diesem Grunde kann vorgesehen sein, dass das obere Nonwoven, bevor es aufgetragen wird, einem verkürzten Airlaidprozess unterzogen wird, bei welchem Fasern auf das obere Nonwoven aufgeblasen werden, umso zu bewirken, dass senkrecht stehende, das Nonwoven durchdringende Fasern eingetragen werden, die den Flüssigkeitsdurchtritt erleichtern.

Das Flächengewicht liegt bevorzugt im Bereich zwischen 2 - 50 g/m², bevorzugt 5 - 20 g/ m², besonders bevorzugt 15 - 18 g/m². Ein solches Nonwoven verbessert den strukturellen Zusammenhalt der Lage insbesondere nach der Aufnahme von Flüssigkeit.

Ferner ist bevorzugt vorgesehen, dass die Superabsorbierenden Fasern, die Zellulosefasern und die Bi-Componentfasern eine Matrix ausbilden. Bevorzugt ist ferner, dass die Superabsorbierenden Partikel in diese Matrix eingebettet sind.

Besonders bevorzugt ist überdies vorgesehen, dass der erfindungsgemäße Wundpflegeartikel einen symmetrischen Schichtaufbau aufweist. Der symmetrische Schichtaufbau führt dabei zu einem im Querschnitt symmetrischen Bild des Wundpflegeartikels. Dies ist für den klinischen Alltag sehr vorteilhaft, weil in diesem Fall das Pflegepersonal den Wundpflegeartikel auf die Wunde aufbringen kann, ohne sich Gedanken über die richtige Orientierung machen zu müssen. Hier besteht ein grundlegender Unterschied zu Hygieneartikeln, wie zum Beispiel Monatsbinden, Inkontinenzeinlagen oder Windeln, die eine klare Polarität aufweisen, dergestalt, dass es stets eine nach außen gewandte Seite sowie eine zum Körper abgewandte Seite gibt.

Der symmetrische Schichtaufbau stellt hohe Anforderungen an den Fertigungsprozess. Wundpflegeartikel der genannten Art werden häufig im so genannten Airlaid-Verfahren hergestellt. Hierbei werden die zu verwendenden Fasern (im vorliegenden Fall Superabsorbierende Fasern und Bi-Componentfasern sowie ggf. Zellulosefasern) über einen Luftstrom von oben auf eine Bahn geblasen. Dabei kommen in der Regel mehrere nacheinander geschaltete Blaseinrichtungen zum Einsatz. Dem Blasprozess wohnt dabei inne, dass die durch die zweite, dritte oder weitere Blaseinrichtung aufgebrachten Fasern die Tendenz aufweisen, nach unten zu wandern. Hierdurch erfolgt eine Umverteilung der Fasern, die die gewünschte Symmetrie beeinträchtigen kann. Daher kann für einen solchen Prozess vorteilhaft sein, die gewünschten Anteile an Fasern in den einzelnen Blaseinrichtungen abweichend von der eigentlich für eine Symmetrie erforderlichen Mengen so zu verändern, dass die nachfolgenden Wanderprozesse der Fasern berücksichtigt werden.

Der erfindungsgemäße Wundpflegeartikel kann ferner mindestens eine flächige Lage aufweisend Zellulosefasern, Schaumstoff, modifizierte Cellulose und/oder Alginate aufweisen.

Der Begriff "Schaumstoff" bezeichnet einen offen- oder geschlossenporigen Schaumstoff, bevorzugt aus Polyurethan.

Bei modifizierter Cellulose handelt es sich bevorzugt um Derivate der Cellulose, bevorzugt Nanocellulose, sulfonierte und/oder sulfoalkylierte Cellulose und deren Derivate, bevorzugt Celluloseethylsulfonate, carboxyalkylierte Cellulose, bevorzugt Carboxymethylzellulose, Carboxyethylcellulose und/oder Carboxypropylcellulose, komplexere Cellulosederivative, wie Sulphoethylcarboxymethylcellulose, Carboxymethylhydroxyethylcellulose, Hydroxy-propylmethylcellulose, und amidierte Cellulosederivate, wie Carboxymethylcellulose-Amid oder Carboxypropylcellulose-Amid. Carboxymethylcellulose liegt insbesondere in Form von Natriumcarboxy-methylcellulose vor und ist unter dem Namen "Hydrofaser" im Handel.

In Hygiene- und Wundprodukten werden die Fasern in eine flächige Matrix überführt. Durch die Aufnahme von Flüssigkeit aus dem Wundexsudat werden die Fasern nach und nach in ein Gelkissen umgewandelt, das die Flüssigkeit hält und nicht wieder freigibt. Dabei sind die Fasern so aufgebaut, dass das Wundexsudat nur in vertikaler Richtung aufgenommen wird. Dies bedeutet dass, solange die Kapazität reicht, das Exsudat nicht über den Wundrand fließt. Auf diese Weise kann eine Wundrandmazeration effektiv verhindert werden. Auch Chitine, Chitosane und deren Derivate sollen im vorliegenden Kontext als Cellulosederivate verstanden werden.

Alginate werden aus den Braunalgen gewonnen und zu einem faserigen Vlies verwoben. Chemisch handelt es sich um Polysaccharide, und zwar Calcium- und/oder Natriumsalze der Alginsäuren. Alginate können bis zum 20-fachen ihres Eigengewichtes an Flüssigkeit aufnehmen, dabei wird das Wundexsudat in die Hohlräume eingelagert. Die im Alginatgitter enthaltenen Ca²⁺ Ionen werden gegen die Na⁺ Ionen aus dem Exsudat ausgetauscht, bis der Sättigungsgrad an Na-Ionen im Alginat erreicht ist. Dabei kommt es zu einem Aufquellen der Wundauflage und zur Umwandlung der Alginatfaser in einen Gelkörper durch Aufquellen der Fasern.

Weiterhin ist bevorzugt vorgesehen, dass der Wundpflegeartikel eine Hülle aufweist, die mindestens partiell aus einem flüssigkeitsdurchlässigen Material besteht. Eine solche Hülle weist eine mannigfaltige Funktion auf. Sie kann u.a. das Verkleben des Wundpflegeartikels mit der Wunde verhindern, einen Rückfluss von Exsudat in die Wunde verhindern, hypoallergen wirken und eine Wundrandmazeration verhindern. Die Hülle ist bevorzugt wenigstens teilweise von einer Naht abgeschlossen, beispielsweise einer Klebenaht oder einer Ultraschallnaht, und kann sowohl aus einer Folie oder einem Film (beispielsweise aus PE) als auch aus einem Nonwoven (beispielsweise aus PP) oder Fleece bestehen.

Insbesondere kann vorgesehen sein, dass die Hülle Poren aufweist, die im Mittel kleiner sind als die Superabsorbierenden Partikel. Auf diese Weise kann ein Herausrieseln von Partikel aus der Hülle vermieden werden. Letzteres kann insbesondere den Sterilisationsprozess behindern, nämlich dann, wenn Partikel in den Bereich der Siegelbarriere gelangen und dort ggf. Undichtigkeiten verursachen.

Die Hülle kann auch mit einem Schwermetall in elementarer oder Ionenform beschichtet oder versetzt sein, beispielsweise Silber, Zink oder Kupfer.

Die Hülle kann auch mit einem Material beschichtet sein, das durch hydrophobe Interaktionen Bakterien bindet, wie beispielsweise Dialkylcarbamoylchlorid (DACC).

Die Porengröße beeinträchtigt jedoch auch die Durchflussgeschwindigkeit des aufzunehmenden Exsudats. Dies trifft insbesondere für mehr oder minder hydrophobe Polymermaterialien zu. Durch eine geeignete Avivage-Behandlung kann allgemein die Benetzbarkeit der Hülle verbessert und dadurch auch die Durchflussgeschwindigkeit auch bei kleinen Poren sichergestellt werden.

Die Poren oder Maschen der Hülle sind bevorzugt 0,05 mm bis 1,0 mm, vorzugsweise 0,20 mm bis 0,50 mm gross. Weiterhin kann bevorzugt vorgesehen sein, dass die Poren oder Maschen durch die Fäden- oder Faserabschnitte begrenzt sind, welche im Schnitt durch die Hülle etwa bogenförmig sind und mit ihren Bogen-Scheiteln nach außen zeigen.

Bevorzugt ist dabei vorgesehen, dass die flächige Lage
(i) in Draufsicht auf ihre Flachseite eine Fläche (F1) aufweist, welche im nicht benetzten Zustand um 3% bis 75% kleiner als die Fläche (F2) des von der Hülle bereitgestellten Innenraums ist, und/oder
(ii) die Hülle mindestens abschnittsweise ein elastisches Material aufweist.

Im erstgenannten Fall ist von einem sogenannten Expansionsraum, die Rede, welchen die Hülle der flächigen Lage zur Verfügung stellt. In beiden Fällen wird so Sorge getragen, dass die Hülle der durch die Aufnahme von Flüssigkeit bedingten Volumenzunahme der flächigen Lage keinen Widerstand entgegensetzt, und letztere so ihre volle Absorptionskapazität ausschöpfen kann. Das elastische Material kann beispielsweise Lycra, Elastan, Polypropylen, Kautschuk, Latex oder ähnliches aufweisen.

Ferner ist bevorzugt vorgesehen, dass die Hülle mindestens partiell aus einem dreidimensionalen Wunddistanzgitter besteht bzw. von diesem be- oder unterlegt ist. Bei besagtem Wunddistanzgitter handelt es sich bevorzugt um ein aus einer Polyethylenfolie durch Blasverfahren erzeugtes Wunddistanzgitter, wie beispielsweise in der EP 2 004 116 A1 beschrieben. Ebenso kann es sich beispielsweise um ein Silikongitter handeln.

Auch ein solches Gitter hat mannigfaltige Funktionen. Es kann, je nach Ausgestaltung der Poren, eine Ventilfunktion ausüben und so den Rückfluss von Exsudat verhindern (bevorzugt bei trichter- oder kragenförmiger Ausgestaltung der Poren). Es kann verhindern, dass der Wundpflegeartikel mit der Wunde verklebt (bevorzugt bei Verwendung eines Silikonmaterials). Es kann bei geeigneter Anordnung abrasive Eigenschaften ausbilden und so die Biofilme auf der Wunde abtragen bzw. deren Entstehung verhindern (bevorzugt bei trichter- oder kragenförmiger Ausgestaltung der Poren). Es kann ferner Blutstillende Eigenschaften aufweisen und ggf. auch imstande sein, Bakterien durch statische Wechselwirkungen zu immobilisieren bzw. zu binden (bevorzugt bei Verwendung eines Polyethylenmaterials oder eines Materials mit positiver Nettoladung). Ferner kann die Oberfläche funktionalisiert sein, beispielsweise mit einer Silber- oder Silikonbeschichtung.

Bevorzugt ist ferner vorgesehen, dass die Hülle mindestens partiell aus einem imprägnierten bzw. wasserundurchlässigen Material besteht bzw. von diesem be- oder unterlegt ist. Hierbei kann es sich um einen farblich ggf. auffällig gestalteten Wäscheschutz ("Backsheet") handeln.

Bevorzugt ist ferner vorgesehen, dass der Wundpflegeartikel einen Anteil an mindestens einem elementaren oder in Ionenform vorliegenden Schwermetall aufweist. Schwermetalle wirken in feinstverteilter Form bakterizid, was aufgrund der großen reaktiven Oberfläche auf die hinreichende Entstehung von löslichen Schwermetallionen zurückzuführen ist.

Durch die Dotierung mit mindestens einem elementaren oder in Ionenform vorliegenden Schwermetall kann dem primärverband eine antibakterielle Wirkung verliehen werden, was Komplikationen bei infektionsgefährdeten Wunden (Dekubitus, Ulcus Cruris, Verbrennungswunden, etc.) vermindert und gleichzeitig die Verweildauer der Wundauflage erhöhen kann.

Bevorzugt ist dabei vorgesehen, dass das mindestens eine elementare oder in Ionenform vorliegenden Schwermetall ausgewählt ist aus der Gruppe enthaltend Kupfer, Zink und/oder Silber. Die oben genannten bakteriziden Eigenschaften gelten in besonderer Form für diese drei Metalle.

Ferner ist bevorzugt vorgesehen, dass die flächige Lage oder die Hülle mindestens auf einer Seite durch eine Abdeckfolie belegt oder ersetzt ist. Bevorzugt weist besagte Abdeckfolie mindestens eine der nachfolgenden Eigenschaften auf:
- Adhäsive Beschichtung
- flüssigkeitsdicht
- wasserdampfdurchlässig und/oder
- elastisch.

Dabei ist besonders bevorzugt vorgesehen, dass die Abdeckfolie über eine Peripherie des Wundpflegeartikels hinausragt und an der Umgebungshaut der Wunde anbringbar ist. Auf diese Weise ist ein sogenanntes Border- oder Island-Dressing verwirklicht.

Alternativ ist vorgesehen, dass die Hülle selbst mindestens auf einer Seite eine adhäsive Beschichtung aufweist. In den genannten Fällen weist die adhäsive Beschichtung bevorzugt einen Acrylatkleber, einen Silikonkleber, einen Stärkekleber, einen Hydrokolloidkleber und/oder einen anderen geeigneten physiologisch unbedenklichen Kleber auf.

Der Wundpflegeartikel kann ferner mindestens einen Bestandteil ausgewählt aus der Gruppe enthaltend
- Hyaluronsäure (bevorzugt als Ummantelung zu superabsorbierenden Polymeren)
- Octenidin
- Dimeticon
- Aktivkohle
aufweisen.

Ferner ist erfindungsgemäß
a) der Wundpflegeartikel gemäß einem der vorhergehenden Ansprüche zur Verwendung in einem Unterdruck-Wundversorgungssystem, und
b) der Wundpflegeartikel gemäß einem der vorhergehenden Ansprüche zur Verwendung bei der Behandlung akuter und postinterventioneller Wunden, chronischer Wunden, diabetischer Wunden oder Druckgeschwüre, Tumorwunden, Verbrennungen, leicht bis stark exsudierender Wunden, sowie tiefer Wunden, die eine Defektauffüllung benötigen,
vorgesehen. Dabei ist insbesondere die Verwendung im Sacralbereich besonders bevorzugt.

Das erfindungsgemäße Vlies kann wie folgt hergestellt werden:

### 1. Dry spinning

10 dtex starke superabsorbierende Fasern aus einem Copolymer der obigen Zusammensetzung werden durch Trockenspinnen aus wässriger Lösung und Zuschneiden (Stapellänge 6 mm) erzeugt, und anschließend bei 200°C vernetzt, bis Esterbrücken zwischen den Carbonsäure-und Hydroxylgruppen ausgebildet werden. Die superabsorbierenden Fasern hatten eine Absorption von 50 g/g (gemessen durch den Free Swell Absorbancy Test) und eine Retention unter Last von 35 g/g.

### 2. Wet laid-Verfahren

Für die Herstellung von wet-laid Vliesstoffen wird ein Pulp Evaluation Apparats von Mavis Manufacturing Company, London verwendet. Alle Vliesstoffe wurden als Bahnen mit 1,2 g Trockengewicht produziert. Die erforderliche Menge an trockener Zellulose (Rayon XF grade) wurde bei 5000 U/min in 2 Liter Wasser mit einem Hochschermischer dispergiert. Die Zellulosefaser wies ein Absorptionsvermögen von weniger als 10 g/g Retention auf, gemessen durch Zentrifugieren einer 0,9 Gew.% Kochsalzlösung. Die superabsorbierende Faser wurde in 100 ml Wasser für ein paar Sekunden dispergiert, bis sie gequollen war, und dann zu der dispergierten Zellulosefaser gegeben. Die Mischung wurde mit einem Spatel gerührt. Die Mischung wurde dann auf eine Säule zur Papierherstellung zugegeben, und das Vlies wurde auf einer 25 mesh Sieb ausgeformt. Die Fasern wurden gepresst und dann bei Raumtemperatur getrocknet. Dabei entstanden Bahnen mit den folgenden Eigenschaften.

| % Superabsorbierende Fasern | | Gewichtsanteil Zellstoff (g) | Gewichtsanteil Superabsorbierende Fasern | Absorption von 0.9 % Koch salzlösung (g/g) unter Last (Free Swell Absorbancy Test) | Retention unter Last (g/g) |
|---|---|---|---|---|---|
| **Control** | **0** | **1.20** | **0** | **9.79** | **8.14** |
| **Example 1** | **5** | **1.14** | **0.06** | **10.59** | **9.48** |
| **Example 2** | **10** | **1,08** | **0.12** | **11.04** | **10.04** |
| **Example 3** | **20** | **0,96** | **0.24** | **12.88** | **10.56** |

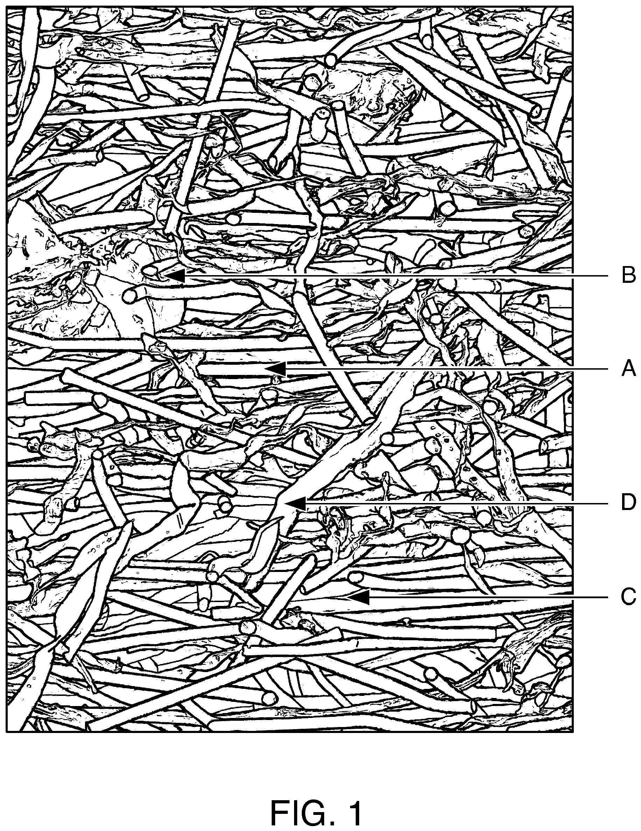

Fig 1. Zeigt ein erfindungsgemäßes Vlies in rasterelektronenmikroskopischer Aufnahme. Dabei bezeichnet A die superabsorbierenden Fasern, B die superabsorbierenden Partikel, und C eine polsternde Lage aus Zellulosefasern und dazwischen liegender Luft ("Fluff Pulp") (D).

## Patentansprüche

1. Wundpflegeartikel aufweisend mindestens eine flächige Lage aus einem Gewebe oder einem Vlies aufweisend
• Superabsorbierende Fasern, und
• Superabsorbierende Partikel, und
wobei die flächige Lage ferner Bi-Component-Fasern aufweist,
und wobei die Bi-Component-Fasern einen Anteil eines thermoplastischen Polymers mit vergleichsweise niedrigem Schmelzpunkt enthalten, das bei Erwärmen schmilzt und so als Heißkleber fungiert.

2. Wundpflegeartikel gemäß Anspruch 1, wobei die Superabsorbierenden Fasern ein quervernetzes Polymer aufweisen, das mindestens aus den Monomerbestandteilen
• Acrylsäure bzw. Acrylat ("AA")
• Methylacrylat bzw. Methylacrylsäure ("MA")
• Partiell zum Natriumsalz neutralisierte Acrylsäure bzw. Methylacrylsäure ("AANa")
hergestellt ist, wobei die Quervernetzungen zwischen den einzelnen Polymerketten als Esterbindungen zwischen den Säuregruppen der Acrylsäure bzw. dem Methylacrylat und dem partiellen Natriumsalz ausgebildet sind.

3. Wundpflegeartikel gemäß einem der vorherigen Ansprüche, wobei die Bi-Component-Fasern durch zwei coextrudierte Polymere in sheath/core-Konfiguration oder side-by-side-Konfiguration gebildet werden.

4. Wundpflegeartikel gemäß einem der vorherigen Ansprüche, wobei die flächige Lage mindestens auf einer Seite von einem dünnen Nonwoven be- oder unterlegt ist.

5. Wundpflegeartikel gemäß einem der vorherigen Ansprüche, wobei die Superabsorbierenden Fasern und/oder die Bi-Componentfasern eine Matrix ausbilden.

6. Wundpflegeartikel gemäß einem der vorherigen Ansprüche, wobei die Superabsorbierenden Partikel in die Matrix aus Fasern eingebettet sind.

7. Wundpflegeartikel gemäß einem der vorherigen Ansprüche, aufweisend einen symmetrischen Schichtaufbau.

8. Wundpflegeartikel gemäß einem der vorherigen Ansprüche, ferner aufweisend mindestens eine flächige Lage aufweisend Zellulosefasern, Schaumstoff, modifizierte Cellulose und/oder Alginate.

9. Wundpflegeartikel gemäß einem der vorherigen Ansprüche, ferner aufweisend eine Hülle, die mindestens partiell aus einem flüssigkeitsdurchlässigen Material besteht.

10. Wundabdeckungsartikel gemäß Anspruch 9, **dadurch gekennzeichnet, dass**
(i) die flächige Lage in Draufsicht auf ihre Flachseite eine Fläche (F1) aufweist, welche im nicht benetzten Zustand um 3% bis 75% kleiner als die Fläche (F2) des von der Hülle bereitgestellten Innenraums ist, und/oder
(ii) die Hülle mindestens abschnittsweise ein elastisches Material aufweist.

11. Wundpflegeartikel gemäß Anspruch -9 oder 10, wobei die Hülle mindestens partiell aus einem dreidimensionalen Wunddistanzgitter besteht bzw. von diesem be- oder unterlegt ist.

12. Wundpflegeartikel gemäß Anspruch 9 - 11, wobei die Hülle mindestens partiell aus einem imprägnierten bzw. wasserundurchlässigen Material besteht bzw. von diesem be- oder unterlegt ist.

13. Wundpflegeartikel gemäß einem der vorherigen Ansprüche, aufweisend einen Anteil an mindestens einem elementaren oder in Ionenform vorliegenden Schwermetall.

14. Wundpflegeartikel gemäß Anspruch 13, wobei das mindestens eine elementare oder in Ionenform vorliegenden Schwermetall ausgewählt ist aus der Gruppe enthaltend Kupfer, Zink und/oder Silber.

15. Wundpflegeartikel nach einem der Ansprüche 8-14 **dadurch gekennzeichnet, dass** die flächige Lage oder die Hülle mindestens auf einer Seite durch eine Abdeckfolie belegt oder ersetzt ist.

16. Wundpflegeartikel gemäß Anspruch 15, **dadurch gekennzeichnet, dass** die Abdeckfolie über eine Peripherie des Wundpflegeartikels hinausragt und an der Umgebungshaut der Wunde anbringbar ist.

17. Wundpflegeartikel nach einem der Ansprüche 8-16 **dadurch gekennzeichnet, dass** die flächige Lage oder die Hülle mindestens auf einer Seite eine adhäsive Beschichtung aufweist.

18. Wundpflegeartikel gemäß einem der vorhergehenden Ansprüche zur Verwendung in einem Unterdruck-Wundversorgungssystem.

19. Wundpflegeartikel gemäß einem Ansprüche 1 bis 17 zur Verwendung bei der Behandlung akuter und postinterventioneller Wunden, chronischer Wunden, diabetischer Wunden oder Druckgeschwüre, Tumorwunden, Verbrennungen, leicht bis stark exsudierender Wunden, sowie tiefer Wunden, die eine Defektauffüllung benötigen.

## Claims

1. Wound care article having at least one planar layer consisting of a woven or non-woven tissue, containing
- super-absorbent fibers and
- super-absorbent particles, and
whereby the planar layer also contains bi-component fibers, and whereby the bi-component fibers contain a portion of a thermoplastic polymer with a comparatively low melting point, which melts when being heated and therefore acts as a hotmelt adhesive.

2. Wound care article according to Claim 1, whereby the super-absorbent fibers contain a cross-linked polymer manufactured at least from the monomer components
- acrylic acid or acrylate, respectively ("AA"),
- methyl acrylate or methacrylic acid ("MA"),
- acrylic acid or methacrylic acid partially neutralized to form the sodium salt ("AANa");
Whereby the cross-linkages between the individual polymer chains being formed as ester bonds between the acid groups of the acrylic acid or the methacrylate, respectively, and the partial sodium salt.

3. Wound care article according to one of the above Claims, the bicomponent fibers being formed by two co-extruded polymers in the sheath/core configuration or the side-by-side configuration.

4. Wound care article according to one of the above Claims, the planar layer being covered or underlaid by a thin non-woven fabric, at least on one side.

5. Wound care article according to one of the above Claims, the super-absorbent fibers and/or the bicomponent fibers forming a matrix.

6. Wound care article according to one of the above Claims, the super-absorbent particles being embedded in the fiber matrix.

7. Wound care article according to one of the above Claims, having a symmetrical layered structure.

8. Wound care article according to one of the above Claims, further having at least one planar layer comprising cellulose fibers, foamed material, modified cellulose and/or alginates.

9. Wound care article according to one of the above Claims, further comprising a sheath which consists at least partially of a liquid-permeable material.

10. Wound covering article according to Claim 9, **characterized in that** (i) in top view, the planar layer has on its flat side a surface (F1) which in the non-wetted state is 3% to 75% smaller than the surface (F2) of the inner space provided by the sheath, and/or
(ii) the sheath contains, at least in parts, an elastic material.

11. Wound care article according to Claim 9 or 10, the sheath at least partially consisting of a three-dimensional protective wound dressing or is covered or supported by it, respectively.

12. Wound care article according to Claims 9-11, the sheath consisting at least partially of an impregnated or water-impermeable material or being covered or supported by it, respectively.

13. Wound care article according to one of the above Claims, containing a portion of at least one heavy metal in elementary or ionic form.

14. Wound care article according to Claim 13, the at least one heavy metal in elementary or ionic form being selected from the group containing copper, zinc and/or silver.

15. Wound care article according to one of Claims 8-14, **characterized in that** the planar layer or the sheath is covered or replaced, at least on one side, by a covering film.

16. Wound care article according to Claim 15, **characterized in that** the covering film extends beyond a periphery of the wound care article and can be attached to the skin surrounding the wound.

17. Wound care article according to one of Claims 8-16, **characterized in that** the planar layer or the sheath has an adhesive coating at least on one side.

18. Wound care article according to one of the above Claims for use in a negative pressure wound therapy system.

19. Wound care article according to one of Claims 1 through 17, for use in the treatment of acute and postinterventional injuries, chronical wounds, diabetic wounds or pressure ulcers, tumor wounds, burns, slightly to heavily exudating wounds as well as deep wounds requiring a filling of the defect.

## Revendications

1. Article pour le soin de plaies présentant au moins une couche plane composée d'un tissé ou d'un non-tissé, présentant
• des fibres super-absorbantes, et
• des particules super-absorbantes, et
dans lequel la couche plane présente en outre des fibres bi-composantes,
et dans lequel les fibres bi-composantes ont une teneur en un polymère thermoplastique présentant un point de fusion comparativement bas, qui fond lorsqu'il est chauffé et sert ainsi de thermocollant.

2. Article pour le soin de plaies selon la revendication 1, dans lequel les fibres super-absorbantes présentent un polymère à réticulation croisée qui est fabriqué au moins à partir des éléments monomères suivants:
• acide acrylique, respectivement acrylate (« AA »)
• acrylate de méthyle, respectivement acide méthacrylique (MA »)
• acide acrylique, respectivement acide méthacrylique, partiellement neutralisé en du sel de sodium (« AANa »),
dans lequel les réticulations croisées entre les différentes chaînes de polymères sont conçues en tant que liaisons d'ester entre les groupes acides de l'acide acrylique, respectivement l'acrylate de méthyle et le sel de sodium partiel.

3. Article pour le soin de plaies selon l'une des revendications précédentes, dans lequel les fibres bi-composantes sont formées par deux polymères coextrudés en configuration gaine/noyau ou côte à côte.

4. Article pour le soin de plaies selon l'une des revendications précédentes, dans lequel la couche plane est revêtue dessus ou dessous par un non-tissé fin sur au moins une face.

5. Article pour le soin de plaies selon l'une des revendications précédentes, dans lequel les fibres super-absorbantes et/ou les fibres bi-composantes forment une matrice.

6. Article pour le soin de plaies selon l'une des revendications précédentes, dans lequel les particules super-absorbantes sont enchâssées dans la matrice de fibres.

7. Article pour le soin de plaies selon l'une des revendications précédentes, présentant un montage en couches symétrique.

8. Article pour le soin de plaies selon l'une des revendications précédentes, présentant en outre au moins une couche plane présentant des fibres de cellulose, un produit alvéolaire, de la cellulose modifiée et/ou des alginates.

9. Article pour le soin de plaies selon l'une des revendications précédentes, présentant en outre une enveloppe qui est composée au moins partiellement d'un matériau perméable aux liquides.

10. Article pour le revêtement de plaies selon la revendication 9, **caractérisé en ce que**
(i) la couche plane présente en vue de dessus sur sa face plane, une surface (F1) qui dans l'état non réticulé, est inférieure de 3 % à 75 % à la surface (F2) de l'intérieur fourni par l'enveloppe, et/ou
(ii) l'enveloppe présente un matériau élastique, au moins par tronçons.

11. Article pour le soin de plaies selon la revendication 9 ou 10, dans lequel l'enveloppe est composée au moins partiellement d'un grillage tridimensionnel à distance par rapport à la plaie ou est revêtue dessus ou dessous par celui-ci.

12. Article pour le soin de plaies selon les revendications 9 - 11, dans lequel l'enveloppe est composée au moins partiellement d'un matériau imprégné, respectivement imperméable à l'eau ou est revêtue dessus ou dessous par celui-ci.

13. Article pour le soin de plaies selon l'une des revendications précédentes, présentant une teneur en au moins un métal lourd élémentaire ou présent sous forme d'ions.

14. Article pour le soin de plaies selon la revendication 13, dans lequel l'au moins un métal lourd élémentaire ou présent sous forme d'ions est sélectionné dans le groupe contenant le cuivre, le zinc et/ou l'argent.

15. Article pour le soin de plaies selon les revendications 8 - 14, **caractérisé en ce que** la couche plane ou l'enveloppe est au moins recouverte ou remplacée sur un côté par un film de revêtement.

16. Article pour le soin de plaies selon la revendication 15, **caractérisé en ce que** le film de revêtement fait saillie au-dessus d'une périphérie de l'article pour le soin de plaies et peut être appliqué sur la peau en contour de plaie.

17. Article pour le soin de plaies selon les revendications 8 - 16, **caractérisé en ce que** la couche plane ou l'enveloppe présente un revêtement adhésif au moins sur un côté.

18. Article pour le soin de plaies selon l'une des revendications précédentes destiné à l'utilisation dans un système de soin de plaies à dépression.

19. Article pour le soin de plaies selon l'une des revendications 1 à 17, destiné à l'utilisation dans le traitement des plaies aiguës et post-interventionnelles, des plaies chroniques, des plaies diabétiques ou des escarres de compression, des plaies de tumeur, des brûlures, des plaies à exsudation légère à forte, ainsi que des plaies profondes qui nécessitent de combler le défaut.
